# EUROPEAN PATENT APPLICATION

(11) **EP 4 756 035 A1**
(43) Date of publication of application: **10.06.2026**
(21) Application number: 24859443.4
(22) Date of filing: 13.08.2024
(51) Int. Cl.: C12Q 1/6806, C12N 15/09, C12N 15/10

(54) **NUCLEIC ACID PURIFICATION METHOD AND KIT FOR NUCLEIC ACID PURIFICATION**

(30) Priority: 01.09.2023 JP 2023142213
(71) Applicant: Eiken Kagaku Kabushiki Kaisha, Tokyo 101-0062 (JP)
(72) Inventor: NAKAGAWA SHIMIZU, Yuki, Shimotsuga-gun, Tochigi 329-0114 (JP)
(74) Representative: Boult Wade Tennant LLP
(86) International application number: PCT/JP2024/028867
(87) International publication number: WO 2025/047407

(57) **Abstract**

An object of the present invention is to provide a method of purifying a nucleic acid from a sample containing a nucleic acid easily, rapidly, and with high purity without using proton-type zeolites, and a kit for such purification. The nucleic acid purification method of the present invention is a method including a step of bringing a sample containing a nucleic acid into contact with an aprotic zeolite.

## Description

### Technical Field

The present invention relates to a nucleic acid purification method and a kit for nucleic acid purification.

### Background Art

Currently, nucleic acid amplification techniques typified as a PCR method and an LAMP method have become widespread in all fields of biology, including molecular biology and medicine, and are widely used in, for example, genetic diagnosis, DNA testing, food inspection, environmental hygiene inspection, and inspections of plants and animals. When nucleic acids in a specimen are amplified, it is generally necessary to extract and purify nucleic acids from the specimen. Examples of methods of extracting and purifying nucleic acids from a specimen include methods using currently commercially available nucleic acid extraction kits. On the other hand, when a general commercially available nucleic acid extraction kit (for example, QIAamp Viral RNA Mini Kit (commercially available from Qiagen)) is used, high-purity nucleic acids can be obtained. However, it requires a plurality of devices such as a high-speed centrifuge and a heating heater, and it is difficult to use the kit unless the operation environment is fully equipped with such devices. In addition, the procedure is complicated because it involves several tens of operation steps, and it takes about 1 hour to extract and purify nucleic acids (for example, Non Patent Literature 1 and 2). In nucleic acid amplification techniques, when there are a large number of test samples, it is desirable to prepare samples that can be applied for nucleic acid amplification in a short time. Therefore, it is difficult to apply extraction and purification methods that require complicated operations. In addition, when more simplified commercially available nucleic acid extraction kits (for example, Kaneka Easy DNA extraction kit (commercially available from Kaneka Corporation), Template Prepper for DNA (commercially available from Nippon Gene Co., Ltd.), and ISOSPIN Viral RNA (commercially available from Nippon Gene Co., Ltd.)) are used, nucleic acids can be extracted and purified in a short time, but the purity of the nucleic acids may be low. In addition, even when such kits are used, a heating operation may be required, and the procedure may involve several tens of operation steps, and thus it is hard to say that nucleic acids can be extracted and purified easily, rapidly, and with high purity. Accordingly, there is a market demand for a method of extracting and purifying nucleic acids easily, rapidly, and with high purity without requiring a large device.

In addition, methods of extracting and purifying nucleic acids from cell lysates and the like using zeolite are also known. For example, Patent Literature 1 discloses a method of preparing a sample for nucleic acid amplification to be used for amplifying nucleic acids contained in a biological sample, including an extraction step of adding a nucleic acid extraction reagent containing an anionic surfactant and/or an alkali to a biological sample to obtain a nucleic acid extraction solution, and a step of bringing the nucleic acid extraction solution into contact with zeolite, wherein the zeolite is a proton-type zeolite, and substances adsorbed to the zeolite are removed. Patent Literature 2 discloses, in a method of isolating nucleic acids from a biological solution (for example, cell lysate) cell lysate, a method of pretreating a clarified lysate using zeolite, and describes that, when zeolite is added to a cell lysate treated with an alkaline reagent such as sodium dodecyl sulfate (SDS), SDS and the like are adsorbed, and the cell lysate can be clarified by centrifugation.

### Citation List

### Patent Literature

[Patent Literature 1] Japanese Patent No. 5290987
[Patent Literature 2] Japanese Unexamined Patent Publication No. 2005-531329

### Non Patent Literature

[Non Patent Literature 1] National Institute of Infectious Diseases, "Manual for the Detection of Pathogens 2019-nCoV Ver. 2. 5," February 15, 2020, https://www.niid.go.jp/niid/images/lab-manual/2019-nCoV20200215.p df
[Non Patent Literature 2] "QIAamp (registered trademark) Viral RNA Mini Protocol and Troubleshooting," (commercially available from QIAGEN), April 2010, https://www.qiagen.com/jp/resources/ download.aspx?id=7d6918b1-77 dd-4dac-b694-a9ca8aa58b43&lang=ja-JP

### Summary of Invention

### Technical Problem

The inventors have found that there are zeolites to which nucleic acids adsorb depending on components of a sample containing nucleic acids, purification conditions and the like, and that such zeolites are proton-type zeolites.

An object of the present invention is to provide a method of purifying nucleic acids from a sample containing nucleic acids easily, rapidly, and with high purity without using proton-type zeolites, and a kit for such purification.

### Solution to Problem

The inventors found that, surprisingly, when a sample containing nucleic acids is mixed with an aprotic zeolite, the adsorption of nucleic acids from the sample to the aprotic zeolite is significantly low, but the adsorption of substances other than nucleic acids is high, and completed the present invention.

Specifically, the present invention relates to, for example, the following inventions.
[1] A nucleic acid purification method, including a step of bringing a sample containing a nucleic acid into contact with an aprotic zeolite.
[2] The purification method according to [1], wherein the sample is at least one specimen selected from the group consisting of blood, cerebrospinal fluid, urine, feces, sputum, saliva, nasal discharge, swab fluid, amniotic fluid, and gargle fluid.
[3] The purification method according to [1] or [2], wherein, in the contacting step, at least a portion of the nucleic acid is not adsorbed to the aprotic zeolite.
[4] The purification method according to any one of [1] to [3], wherein the aprotic zeolite is at least one selected from the group consisting of a ferrierite-type zeolite, a ZSM-5-type zeolite, a Y-type zeolite, and a beta-type zeolite.
[5] The purification method according to [4], wherein the aprotic zeolite is at least one selected from the group consisting of a ferrierite-NH₄-type zeolite, a ferrierite-K-type zeolite, a ZSM-5-NH₄-type zeolite, a Y-Na-type zeolite, and a beta-NH₄-type zeolite.
[6] The purification method according to any one of [1] to [5], further including, before the contacting step, a step of obtaining a sample containing the nucleic acid by mixing the specimen with a nucleic acid extraction reagent containing a surfactant and/or by heating the specimen.
[7] The purification method according to [6], wherein the surfactant includes an anionic surfactant.
[8] The purification method according to [7], wherein the anionic surfactant is dodecyl sulfate.
[9] The purification method according to any one of [1] to [8], wherein the nucleic acid is RNA and/or DNA.
[10] A kit for purifying a nucleic acid, including an aprotic zeolite.
[11] The kit according to [10], wherein the aprotic zeolite is at least one selected from the group consisting of a ferrierite-type zeolite, a ZSM-5-type zeolite, a Y-type zeolite, and a beta-type zeolite.
[12] The kit according to [11], wherein the aprotic zeolite is at least one selected from the group consisting of a ferrierite-NH₄-type zeolite, a ferrierite-K-type zeolite, a ZSM-5-NH₄-type zeolite, a Y-Na-type zeolite, and a beta-NH₄-type zeolite.
[13] The kit according to any one of [10] to [12], wherein the kit is a kit for purifying a nucleic acid derived from at least one specimen selected from the group consisting of blood, cerebrospinal fluid, urine, feces, sputum, saliva, nasal discharge, swab fluid, amniotic fluid, and gargle fluid, and further includes a nucleic acid extraction reagent containing a surfactant.
[14] The kit according to [13], wherein the surfactant includes an anionic surfactant.
[15] The kit according to [14], wherein the anionic surfactant is dodecyl sulfate.

### Advantageous Effects of Invention

According to the present invention, it is possible to provide a method of extracting and purifying a nucleic acid from a sample containing a nucleic acid easily, rapidly, and with high purity by efficiently removing substances other than a nucleic acid, and a kit for such extraction and purification. Particularly, according to the present invention, it is possible to extract and purify a nucleic acid from a sample containing a nucleic acid easily, rapidly, and with high purity without using a plurality of devices such as a high-speed centrifuge and a heating heater. In addition, since the aprotic zeolite exhibits significantly low adsorption of a nucleic acid from the sample, the present invention is expected to enable extraction and purification of a nucleic acid in a high yield. In addition, the present invention is highly versatile because it can be applied to purification of either or both of DNA and RNA.

In addition, as described above, according to the present invention, it is possible to purify a nucleic acid easily, rapidly, and with high purity. Therefore, it is possible to obtain an effective sample to be subjected to a nucleic acid amplification reaction, and highly sensitive measurement using nucleic acid amplification techniques becomes possible.

### Description of Embodiments

Hereinafter, forms for implementing the present invention will be described in detail. However, the present invention is not limited to the following embodiments.

### [Nucleic acid purification method]

A nucleic acid purification method according to the present embodiment includes a step of bringing a sample containing a nucleic acid into contact with an aprotic zeolite (contacting step). As will be shown in examples below, the aprotic zeolite does not adsorb at least a portion of a nucleic acid in a sample containing a nucleic acid, but adsorbs substances other than a nucleic acid. Therefore, when the sample containing a nucleic acid is brought into contact with the aprotic zeolite, it is possible to remove substances other than a nucleic acid from the sample containing a nucleic acid, and purify a nucleic acid easily, rapidly, and with high purity.

### <Sample containing nucleic acid>

The sample containing a nucleic acid is an aqueous solution or aqueous suspension in which a nucleic acid and substances other than a nucleic acid are dissolved or suspended. The type of a nucleic acid contained in the sample is not particularly limited, and may be DNA or RNA, and may be single-stranded or double-stranded. The origin of a nucleic acid is not limited, and for example, a nucleic acid may be derived from animals, plants, fungi, bacteria, or viruses. The method of the present invention can particularly purify RNA easily, rapidly, and with high purity. The pH of the sample containing a nucleic acid is not particularly limited.

Examples of substances other than a nucleic acid include proteins (for example, enzymes, glycoproteins, polypeptides, etc.), lipids, sugars (for example, monosaccharides, disaccharides, oligosaccharides, polysaccharides, etc.), salts and the like.

In addition, the sample containing a nucleic acid may be derived from, for example, a specimen obtained from a living organism, or may be derived from a specimen obtained from the environment such as water, soil, or air. The sample containing a nucleic acid is preferably derived from a specimen obtained from a living organism, and more preferably derived from at least one specimen selected from the group consisting of blood (whole blood, plasma, or serum), cerebrospinal fluid, urine, feces, sputum, saliva, nasal discharge, swab fluid, amniotic fluid, and gargle fluid. The sample containing a nucleic acid may be a diluted solution/suspension of the specimen or an extraction solution obtained according to an extraction step described below. The substances other than a nucleic acid may be components other than a nucleic acid derived from these specimens.

The sample containing a nucleic acid may contain a nucleic acid extraction reagent including a surfactant to be described below, and may also contain, as necessary, an inhibitor against substances that decompose a nucleic acid. Examples of such inhibitors include a nuclease inhibitor.

### <Zeolite>

"Zeolite" is a general term for aluminosilicates having micropores in their crystals, and having a three-dimensional network structure as a basic framework, in which SiO₄ and AlO₄ tetrahedrons are bonded by sharing oxygen atoms. Zeolites contain cations in their crystals, for example, alkali metal ions, alkaline earth metal ions, ammonium ions, hydrogen ions (protons) and the like. "Proton-type zeolite" is a zeolite that contains hydrogen ions as cations in its crystal. "Aprotic zeolite" is a zeolite other than a proton-type zeolite. The aprotic zeolite in the purification method according to the present embodiment does not adsorb at least portion of a nucleic acid in the sample containing a nucleic acid.

The crystal structure of the aprotic zeolite is not particularly limited, and examples thereof include A-type, ferrierite-type, MCM-22-type, ZSM-5-type, mordenite-type, L-type, Y-type, X-type, and beta-type. The aprotic zeolite is preferably at least one selected from the group consisting of a ferrierite-type zeolite, a ZSM-5-type zeolite, a Y-type zeolite, and a beta-type zeolite.

The cations in the crystals of the aprotic zeolite are not particularly limited as long as they are other than hydrogen ions. Examples of such cations include alkali metal ions (for example, sodium ions (Na⁺), potassium ions (K⁺), etc.), alkaline earth metal ions (for example, magnesium ions (Mg²⁺), calcium ions (Ca²⁺), etc.), and ammonium ions (NH₄⁺).

The aprotic zeolite is particularly preferably at least one selected from the group consisting of a ferrierite-NH₄-type zeolite, a ferrierite-K-type zeolite, a ZSM-5-NH₄-type zeolite, a Y-Na-type zeolite, and a beta-NH₄-type zeolite. For example, "ferrierite-NH₄-type zeolite" is a zeolite having a ferrierite-type crystal structure and containing ammonium ions as cations.

### <Contacting step>

In the contacting step, a sample containing a nucleic acid is brought into contact with an aprotic zeolite to adsorb substances other than a nucleic acid. Therefore, in the purification method according to the present embodiment, it is possible to purify a nucleic acid from the sample containing a nucleic acid according to the contacting step. "Purification" in this specification means increasing the purity of a nucleic acid from a sample containing a nucleic acid.

In the contacting step, "bring into contact" may mean mixing a sample containing a nucleic acid with an aprotic zeolite, and for example, a sample containing a nucleic acid may be added to a container containing an aprotic zeolite, or an aprotic zeolite may be added to a sample containing a nucleic acid, and the order in which a sample containing a nucleic acid and an aprotic zeolite are brought into contact with each other is not particularly limited.

In the contacting step, the amount of the zeolite per 1 mL of the sample containing a nucleic acid may be, for example, 50 mg or more, 60 mg or more, 70 mg or more, 80 mg or more, 90 mg or more, 100 mg or more, 110 mg or more, or 120 mg or more. In the contacting step, the amount of the zeolite per 1 mL of the sample containing a nucleic acid may be, for example, 350 mg or less, 340 mg or less, 330 mg or less, 320 mg or less, 310 mg or less, or 300 mg or less.

In the contacting step, the contact time is not particularly limited, and may be, for example, 10 seconds to 10 minutes, 20 seconds to 10 minutes, or 30 seconds to 5 minutes.

According to the contacting step, at least some substances other than a nucleic acid in the sample containing a nucleic acid are adsorbed to the aprotic zeolite. "Adsorption" refers to the state in which a substance is bound to the surface or entrapped within the pores of the aprotic zeolite, and the adsorbed substance is removed together when the aprotic zeolite is removed. According to the contacting step, at least 30% or more, at least 40% or more, at least 50% or more, at least 60% or more, at least 70% or more, at least 80% or more, or at least 90% or more of substances other than a nucleic acid in the sample containing a nucleic acid are adsorbed to the aprotic zeolite. In addition, the expression "at least some a nucleic acid are not adsorbed to the aprotic zeolite" may refer to a situation in which, for example, when a sample containing only a nucleic acid and a sample obtained after the sample containing only a nucleic acid is brought into contact with an aprotic zeolite are each subjected to nucleic acid amplification under the same conditions, the difference in the number of nucleic acid copies detected is 25% or less, 20% or less, 15% or less, 10% or less, 5% or less, or 1% or less, and preferably 0% relative to the number of nucleic acid copies detected in the former sample, or it may be equal to or greater than the number of nucleic acid copies detected when nucleic acid amplification is performed using the sample containing only a nucleic acid.

### <Extraction step>

The purification method according to the present embodiment may include, before the contacting step, a step of obtaining a sample containing the nucleic acid by mixing the specimen with a nucleic acid extraction reagent containing a surfactant and/or by heating the specimen (extraction step). Due to the surfactant or the heat treatment, cell membranes, virus envelopes and the like in the specimen are destroyed. Therefore, the nucleic acid can be dissolved or suspended in the sample containing a nucleic acid. When both mixing the specimen with the nucleic acid extraction reagent containing a surfactant and heating the specimen are performed, the order of these steps is not particularly limited. When the purification method according to the present embodiment includes the extraction step, the purification method according to the present embodiment can be considered as an extraction method.

The surfactant is not particularly limited, and may be an ionic surfactant (anionic surfactant, cationic surfactant), a nonionic surfactant, or an amphoteric surfactant, and in order to more easily destroy cell membranes, virus envelopes and the like, an ionic surfactant is preferable, and an anionic surfactant is more preferable. The nucleic acid extraction reagent may contain one type of surfactant or two or more types of surfactants.

Examples of anionic surfactants include higher fatty acid salts, linear alkylbenzene sulfonates, α-sulfo fatty acid methyl ester salts, α-olefin sulfonates, alkylsulfate ester salts, and polyoxyethylene alkylsulfate ester salts, and alkylsulfate ester salts are preferable, and dodecyl sulfates such as lithium dodecyl sulfate (LDS) and sodium dodecyl sulfate (SDS) and sodium cholate are more preferable.

Examples of cationic surfactants include alkyltrimethylammonium salts, dialkyldimethylammonium salts, alkyldimethylbenzylammonium salts, and benzyltrimethylammonium salts, and may include benzalkonium chloride and the like.

Examples of nonionic surfactants include sorbitan fatty acid esters, sucrose fatty acid esters, polyoxyethylene fatty acid esters, polyoxyethylene sorbitan fatty acid esters, polyoxyethylene alkyl ethers, and polyoxyalkylphenyl ethers, and may include TRITON (registered trademark)-X100, Tween (registered trademark) 40 and the like.

Examples of amphoteric surfactants include alkylamino fatty acid salts, alkyl betaines, and alkyl amine oxides, and may include 3-[(3-cholamidopropyl)dimethylammonio]-2-hydroxy-1-propanesulfon ate (CHAPSO), 3-[(3-cholamidopropyl)dimethylammonio]-1-propanesulfonate (CHAPS) and the like.

The content of the surfactant in the nucleic acid extraction reagent may be a general content that allows a nucleic acid to be extracted, and may vary depending on the type of surfactant. The content of the surfactant based on a total amount of the nucleic acid extraction reagent may be, for example, 0.01 w/v% or more, 0.1 w/v% or more, 0.5 w/v% or more, or 1 w/v% or more. The content of the surfactant in the nucleic acid extraction reagent based on a total amount of the nucleic acid extraction reagent may be, for example, 5 w/v% or less, 4 w/v% or less, or 3 w/v% or less. Here, when the nucleic acid extraction reagent contains a plurality of types of surfactants, the content of the surfactant in the nucleic acid extraction reagent is the total content of the plurality of types of surfactants.

The nucleic acid extraction reagent may contain an alkali in addition to the surfactant. Examples of alkalis include sodium hydroxide, potassium hydroxide, lithium hydroxide, calcium hydroxide, and aqueous ammonia, and sodium hydroxide is preferable. The nucleic acid extraction reagent may contain one type of alkali or two or more types of alkali.

When the alkali is sodium hydroxide, the content of the alkali in the nucleic acid extraction reagent based on a total amount of the nucleic acid extraction reagent may be, for example, 10 mM or more, 25 mM or more, 50 mM or more, 75 mM or more, 100 mM or more, 125 mM or more, 150 mM or more, 175 mM or more, or 200 mM or more. When the alkali is sodium hydroxide, the content of the alkali in the nucleic acid-containing reagent based on a total amount of the nucleic acid extraction reagent may be, for example, 500 mM or less, 480 mM or less, 460 mM or less, 440 mM or less, 420 mM or less, or 400 mM or less.

The pH of the nucleic acid extraction reagent is not particularly limited as long as it is a general pH at which a nucleic acid do not decompose, and may be, for example, 5 to 9, 5 to 7, or 6 to 7.

The heat treatment is not particularly limited as long as a nucleic acid can be extracted from a specimen and the nucleic acid can be dissolved or suspended in a sample containing a nucleic acid, and for example, a specimen may be heated using a heat block or the like. The heat treatment temperature may be, for example, 50°C to 200°C, 60°C to 150°C, or 70°C to 100°C. The heat treatment time may be, for example, 1 minute to 30 minutes, 5 minutes to 30 minutes, or 15 minutes to 30 minutes.

### <Removal step>

The purification method according to the present embodiment may include a step of removing the aprotic zeolite (removal step) after the contacting step. In the removal step, the aprotic zeolite is removed to obtain a sample containing purified a nucleic acid.

The aprotic zeolite can be removed, for example, by subjecting the mixture of the sample containing a nucleic acid and the aprotic zeolite after the contacting step to centrifugation, filtration, or a combination thereof. Removal by centrifugation can be easily performed using, for example, a commercially available spin column.

The sample containing purified a nucleic acid obtained in the removal step can be used, for example, in nucleic acid amplification analysis.

[Nucleic acid amplification method] A nucleic acid amplification method according to the present embodiment includes a step of purifying a nucleic acid by performing the purification method according to the present embodiment, and a step of performing a nucleic acid amplification reaction on the purified a nucleic acid (nucleic acid amplification step).

### <Nucleic acid amplification step>

The nucleic acid amplification step can be performed according to a conventional method. More specifically, it can be performed by incubating a reaction solution containing the purified nucleic acid, a primer set containing a base sequence specific to the nucleic acid, a nucleic acid synthesis enzyme such as a DNA polymerase, deoxynucleoside triphosphates (dNTPs: dATP, dTTP, dCTP, and dGTP), a reverse transcriptase as necessary, a labeled probe for detecting an amplification product and the like.

When the nucleic acid is DNA, the nucleic acid amplification reaction may be, for example, a polymerase chain reaction (PCR), a loop-mediated isothermal amplification (LAMP) reaction, a nicking endonuclease amplification reaction (NEAR), a transcription-reverse transcription concerted (TRC) reaction, a whole genome amplification (WGA) reaction, a strand displacement amplification (SDA) reaction, a helicase-dependent amplification (HDA) reaction, a recombinase polymerase amplification (RPA) reaction, or an isothermal chimeric primer-initiated amplification (ICAN) reaction. When the nucleic acid is RNA, the nucleic acid amplification reaction may be a reverse transcription PCR (RT-PCR), a reverse transcription LAMP (RT-LAMP) reaction, a transcription-mediated amplification (TMA) reaction, or a nucleic acid sequence-based amplification (NASBA) reaction.

[Kit for purifying nucleic acid] A kit for purifying a nucleic acid according to the present embodiment (hereinafter referred to as a "purification kit according to the present embodiment") includes an aprotic zeolite. When the purification kit according to the present embodiment is used, it is possible to purify a nucleic acid from the sample containing a nucleic acid. The sample containing a nucleic acid and the aprotic zeolite are as described above.

The purification kit according to the present embodiment may include a nucleic acid extraction reagent containing a surfactant when a specimen-derived sample is used as the sample containing a nucleic acid. The nucleic acid extraction reagent containing a surfactant is as described above.

The purification kit according to the present embodiment may include, in addition to the aprotic zeolite and the nucleic acid extraction reagent containing a surfactant, a buffer solution, a nuclease inhibitor, a device used for purifying a nucleic acid (for example, a spin column, etc.) and the like.

### [Kit for nucleic acid amplification]

A kit for nucleic acid amplification according to the present embodiment (hereinafter referred to as a "nucleic acid amplification kit according to the present embodiment") includes an aprotic zeolite, a DNA polymerase, and deoxynucleoside triphosphates.

The nucleic acid amplification kit according to the present embodiment may include, in addition to the components provided in the purification kit according to the present embodiment, known reagents for performing various nucleic acid amplification reactions. When the target nucleic acid is RNA, the nucleic acid amplification kit according to the present embodiment may further include a reverse transcriptase. Examples of other known reagents for performing various nucleic acid amplification reactions include a buffer solution that provides conditions suitable for enzymatic reactions, enzymes such as dithiothreitol (DTT), protecting agents for stabilizing target nucleic acid sequences, labeled probes for detecting amplification products, and intercalators. In addition, the nucleic acid amplification kit according to the present embodiment may include a device used for detecting nucleic acid amplification.

### [Examples]

Hereinafter, the present invention will be described in more detail with reference to examples. However, the present invention is not limited thereto.

### [Test Example 1: Confirmation of RNA adsorption to various zeolites]

480 mg of the zeolite described below was added to 1,000 µL of a solution in which SARS-CoV-2 transcription RNA was added to a nucleic acid extraction reagent (0.5% sodium dodecyl sulfate (hereinafter referred to as "SDS")), and the mixture was mixed by inversion. The zeolites used are commercially available from Tosoh Corporation: model numbers 930NHA (beta-NH₄ type), 940NHA (beta-NH₄ type), 840NHA (ZSM-5-NH₄ type), 720NHA (ferrierite-NH₄ type), 720KOA (ferrierite-K type), 690HOA (mordenite-H type), 320NAA (Y-Na type), and 320HOA (Y-H type). The suspension mixed by inversion was passed through a 0.45 µm filter, the zeolite and substances adsorbed thereon were removed, and the obtained filtrates were used as zeolite-treated samples 1-1 to 1-8. The target nucleic acid (cDNA of SARS-CoV-2 transcription RNA) was amplified using 5 µL of the zeolite-treated sample and 20 µL of a quantitative PCR reaction reagent according to a quantitative PCR method. In addition, as a positive control, a sample 1-9 in which SARS-CoV-2 transcription RNA was added to a nucleic acid extraction reagent (0.5% SDS) but which was not treated with a zeolite was also prepared, and subjected to target nucleic acid amplification according to the quantitative PCR method in the same manner.

Amplification and detection of the target nucleic acid according to the quantitative PCR method were performed using the primer and probe set shown in Table 1, according to the reaction under the temperature and time shown in Table 3 in the quantitative PCR reaction reagent solution having the composition shown in Table 2. The fluorescence value, which increases as a result of target nucleic acid amplification, was measured in real time using a Thermal Cycler Dice (registered trademark) Real Time System III. Here, the probe composed of a base sequence shown in SEQ ID NO 3 in Table 1 was labeled with FAM at the 5' end and labeled with BHQ1 at the 3' end. The results are shown in Table 4. Here, in Table 4, samples for which no Ct value was detected within the measurement time (40 cycles) are indicated as "N. D.". Here, in Table 4, the "proportion of copies" is the percentage relative to the number of copies of the "no zeolite treatment" sample (defined as 100%).

**[Table 1]**

| <Primer and probe set for quantitative PCR method targeting SARS-CoV-2 RNA as nucleic acid> | |
|---|---|
| Name | Sequence (5'→3') |
| Forward primer | AAATTTTGGGGACCAGGAAC (SEQ ID NO 1) |
| Reverse primer | TGGCAGCTGTGTAGGTCAAC (SEQ ID NO 2) |
| Probe | FAM-ATGTCGCGCATTGGCATGGA (SEQ ID NO 3)-BHQ1 |

**[Table 2]**

| <Composition of quantitative PCR reaction reagent (20 µL)> |
|---|
| 1x One Step PrimeScript III RT-qPCR Mix |
| 0.4 µM forward primer |
| 0.4 µM reverse Primer |
| 0.2 µM probe |
| Distilled water |

**[Table 3]**

| <Reaction conditions> | | |
|---|---|---|
| Step 1 | 52°C 5 min, 95°C 10 sec | 1 cycle |
| Step 2 | 95°C 5 sec→60°C 30 sec | 40 cycles |

**[Table 4]**

| Sample No. | Zeolite model number | Crystal structure | Cation | Number of copies (copies/test) | Proportion of copies |
|---|---|---|---|---|---|
| 1-1 | 930NHA | beta type | NH₄ | 3,410 | 90% |
| 1-2 | 940NHA | beta type | NH₄ | 3,316 | 87% |
| 1-3 | 840NHA | ZSM-5 type | NH₄ | 3,545 | 93% |
| 1-4 | 720NHA | ferrierite type | NH₄ | 3,113 | 82% |
| 1-5 | 720KOA | ferrierite type | K | 3,517 | 92% |
| 1-6 | 690HOA | mordenite type | H | N.D. | - |
| 1-7 | 320NAA | Y type | Na | 3,705 | 97% |
| 1-8 | 320HOA | Y type | H | N.D. | - |
| 1-9 | no zeolite treatment | | | 3,803 | 100% |

In the samples treated with the proton-type zeolite (samples 1-6 and 1-8), target nucleic acid amplification was not detected. This suggests that the proton-type zeolite adsorbed nucleic acids, particularly RNA. On the other hand, the samples treated with the aprotic zeolite (samples 1-1 to 1-5, and 1-7) exhibited target nucleic acid amplification and the number of copies comparable to those of the sample not treated with the zeolite (sample 1-9). This suggests that the aprotic zeolite exhibited significantly low adsorption of nucleic acids, particularly RNA.

### [Test Example 2: Confirmation of DNA adsorption to various zeolites]

Zeolite-treated samples 2-1 to 2-9 were obtained in the same manner as in Test Example 1 except that DNA of *Saccharomyces cerevisiae* (hereinafter referred to as *"S. cerevisiae"*) was added to a nucleic acid extraction reagent (0.5% SDS). In addition, the samples 2-1 to 2-9 were subjected to target nucleic acid (*S. cerevisiae* DNA) amplification according to the quantitative PCR method in the same manner as in Test Example 1 except that the reaction was performed using the primer and probe set shown in Table 5, and the quantitative PCR reaction reagent solution shown in Table 6, under the temperature and time shown in Table 7. In addition, as a positive control, a sample 2-10 in which S. cerevisiae DNA was added to a nucleic acid extraction reagent but which was not treated with a zeolite was also prepared, and subjected to target nucleic acid amplification according to the quantitative PCR method in the same manner. Here, the probe composed of a base sequence shown in SEQ ID NO 6 in Table 5 was labeled with FAM at the 5' end and labeled with BHQ1 at the 3' end. The results are shown in Table 8. Here, in Table 8, samples for which no Ct value was detected within the measurement time (40 cycles) are indicated as 'N. D.". Here, in Table 8, the "proportion of copies" is the percentage relative to the number of copies of the "no zeolite treatment" sample (defined as 100%).

**[Table 5]**

| <Primer and probe set for quantitative PCR method targeting S. *cerevisiae* DNA as nucleic acid> | |
|---|---|
| Name | Sequence (5'→3') |
| Forward primer | GGACTCTGGACATGCAAGAT (SEQ ID NO 4) |
| Reverse primer | ATACCCTTCTTAACACCTGGC (SEQ ID NO 5) |
| Probe | FAM-CCCTTCAGAGCGTTTTCTCTAAATTGATAC (SEQ ID NO 6)-BHQ1 |

**[Table 6]**

| <Composition of quantitative PCR reaction reagent (20 µL)> |
|---|
| 1x Premix Ex Taq (Probe qPCR) |
| 0.5 µM forward primer |
| 0.5 µM reverse primer |
| 0.2 µM probe |
| Distilled water |

**[Table 7]**

| <Reaction conditions> | | |
|---|---|---|
| Step 1 | 95°C 30 sec | 1 cycle |
| Step 2 | 95°C 5 sec→60°C 30 sec | 40 cycles |

**[Table 8]**

| Sample No. | Model number | Crystal structure of zeolite | Cation of zeolite | Number of copies (copies/test) | Proportion of copies |
|---|---|---|---|---|---|
| 2-1 | 930NHA | beta type | NH₄ | 2,291 | 85% |
| 2-2 | 940NHA | beta type | NH₄ | 2,149 | 80% |
| 2-3 | 840NHA | ZSM-5 type | NH₄ | 2,851 | 106% |
| 2-4 | 720NHA | ferrierite type | NH₄ | 2,540 | 94% |
| 2-5 | 720KOA | ferrierite type | K | 2,622 | 97% |
| 2-6 | 722HOA | ferrierite type | H | 479 | 18% |
| 2-7 | 690HOA | mordenite type | H | N.D. | - |
| 2-8 | 320NAA | Y type | Na | 3,034 | 112% |
| 2-9 | 320HOA | Y type | H | N.D. | - |
| 2-10 | no zeolite treatment | | | 2,698 | 100% |

Similar results were obtained with RNA as with DNA. In the samples treated with the proton-type zeolite (samples 2-6 to 2-7 and 2-9), target nucleic acid amplification was not detected, or if detected, the number of copies was very low. This suggests that the proton-type zeolite adsorbed nucleic acids, particularly DNA. On the other hand, the samples treated with the aprotic zeolite (samples 2-1 to 2-5, and 2-8) exhibited target nucleic acid amplification and the number of copies comparable to those of the sample not treated with the zeolite (sample 2-10). This suggests that the aprotic zeolite exhibited significantly low adsorption of nucleic acids, particularly DNA.

### [Test Example 3: Confirmation of effective pH range for RNA adsorption to various zeolites]

Zeolite-treated samples 3-2 and 3-3, 3-5 and 3-6, 3-8 and 3-9, 3-11 and 3-12, 3-14 and 3-15, and 3-17 and 3-18 were obtained in the same manner as in Test Example 1 except that 480 mg of a zeolite was added to 1,000 µL of a solution in which SARS-CoV-2 transcription RNA was added to a nucleic acid extraction reagent (0.5% SDS) adjusted to a pH of 3 to 12, and the mixture was mixed by inversion, and the zeolites used were model numbers 720NHA and 690HOA (commercially available from Tosoh Corporation). In addition, in the same manner as in Test Example 1, the zeolite-treated samples were subjected to target nucleic acid (cDNA of SARS-CoV-2 transcription RNA) amplification according to the quantitative PCR method. In addition, in the same manner as in Test Example 1, as positive controls, samples 3-1, 3-4, 3-7, 3-10, 3-13, and 3-16 in which SARS-CoV-2 transcription RNA was added to a nucleic acid extraction reagent but which were not treated with a zeolite were also prepared, and subjected to target nucleic acid amplification according to the quantitative PCR method in the same manner as in Test Example 1. The results are shown in Table 9. Here, in Table 9, the "proportion of copies" is the percentage relative to the number of copies of the "no zeolite treatment" sample (defined as 100%) in the extraction solution at each pH.

**[Table 9]**

| Sample No. | pH of nucleic acid extraction reagent | Zeolite | Number of copies (copies/test) | Proportion of copies |
|---|---|---|---|---|
| 3-1 | pH 3 | no zeolite treatment | 17,533 | 100% |
| 3-2 | | aprotic zeolite | 17,140 | 98% |
| 3-3 | | proton-type zeolite | 342 | 2% |
| 3-4 | pH 4 | no zeolite treatment | 18,707 | 100% |
| 3-5 | | aprotic zeolite | 20,292 | 108% |
| 3-6 | | proton-type zeolite | 501 | 3% |
| 3-7 | pH 7 | no zeolite treatment | 19,297 | 100% |
| 3-8 | | aprotic zeolite | 22,925 | 119% |
| 3-9 | | proton-type zeolite | 7,622 | 39% |
| 3-10 | pH 10 | no zeolite treatment | 20,388 | 100% |
| 3-11 | | aprotic zeolite | 24,060 | 118% |
| 3-12 | | proton-type zeolite | 16,287 | 80% |
| 3-13 | pH 11 | no zeolite treatment | 18,709 | 100% |
| 3-14 | | aprotic zeolite | 18,808 | 101% |
| 3-15 | | proton-type zeolite | 16,250 | 87% |
| 3-16 | pH 12 | no zeolite treatment | 15,317 | 100% |
| 3-17 | | aprotic zeolite | 18,613 | 122% |
| 3-18 | | proton-type zeolite | 15,481 | 101% |

As shown in Table 9, the samples using a nucleic acid extraction reagent adjusted to a pH of 3 to 7 and treated with the proton-type zeolite exhibited a very low number of target nucleic acid copies compared to the positive control sample not treated with the zeolite. This suggests that, when an acidic to neutral nucleic acid extraction reagent was used, the proton-type zeolite adsorbed RNA. On the other hand, all samples treated with the aprotic zeolite exhibited the number of copies comparable to those of the positive control sample not treated with the zeolite. Therefore, it was speculated that the aprotic zeolite exhibited significantly low adsorption of RNA over any pH range.

### [Test Example 4: Confirmation of effective pH range for DNA adsorption to various zeolites]

Zeolite-treated samples 4-2 and 4-3, 4-5 and 4-6, 4-8 and 4-9, 4-11 and 4-12, 4-14 and 4-15, and 4-17 and 4-18 were obtained in the same manner as in Test Example 1 except that 480 mg of a zeolite was added to 1,000 µL of a solution in which DNA of S. cerevisiae was added to a nucleic acid extraction reagent (0.5% SDS) adjusted to a pH of 3 to 12, and the mixture was mixed by inversion, and the zeolites used were model numbers 720NHA and 690HOA (commercially available from Tosoh Corporation). In addition, in the same manner as in Test Example 2, the zeolite-treated samples were subjected to target nucleic acid amplification according to the quantitative PCR method. In addition, as positive controls, in the same manner as in Test Example 2, samples 4-1, 4-4, 4-7, 4-10, 4-13, and 4-16 in which DNA of S. cerevisiae was added to a nucleic acid extraction reagent but which were not treated with a zeolite were also prepared, and subjected to target nucleic acid amplification according to the quantitative PCR method in the same manner. The results are shown in Table 10.Here, in Table 10, the "proportion of copies" is the percentage relative to the number of copies of the "no zeolite treatment" sample (defined as 100%) in the extraction solution at each pH.

**[Table 10]**

| Sample No. | pH of nucleic acid extraction reagent | Zeolite | Number of copies (copies/test) | Proportion of copies |
|---|---|---|---|---|
| 4-1 | pH 3 | no zeolite treatment | 1,988 | 100% |
| 4-2 | | aprotic zeolite | 2,404 | 121% |
| 4-3 | | proton-type zeolite | 37 | 2% |
| 4-4 | pH 4 | no zeolite treatment | 2,117 | 100% |
| 4-5 | | aprotic zeolite | 2,463 | 116% |
| 4-6 | | proton-type zeolite | 7 | 0% |
| 4-7 | pH 7 | no zeolite treatment | 1,905 | 100% |
| 4-8 | | aprotic zeolite | 2,604 | 137% |
| 4-9 | | proton-type zeolite | 10 | 1% |
| 4-10 | pH 10 | no zeolite treatment | 1,926 | 100% |
| 4-11 | | aprotic zeolite | 2,197 | 114% |
| 4-12 | | proton-type zeolite | 2,030 | 105% |
| 4-13 | pH 11 | no zeolite treatment | 1,807 | 100% |
| 4-14 | | aprotic zeolite | 2,292 | 127% |
| 4-15 | | proton-type zeolite | 2,044 | 113% |
| 4-16 | pH 12 | no zeolite treatment | 1,945 | 100% |
| 4-17 | | aprotic zeolite | 2,160 | 111% |
| 4-18 | | proton-type zeolite | 2,129 | 109% |

Similar results were obtained with RNA as with DNA. The samples using a nucleic acid extraction reagent adjusted to a pH of 3 to 7 and treated with the proton-type zeolite exhibited a very low number of target nucleic acid copies compared to the positive control sample not treated with the zeolite. This suggests that, when an acidic to neutral nucleic acid extraction reagent was used, the proton-type zeolite adsorbed DNA. On the other hand, all samples treated with the aprotic zeolite exhibited the number of copies comparable to that of the positive control samples not treated with the zeolite. Therefore, it was speculated that the aprotic zeolite exhibited significantly low adsorption of DNA over any pH range.

### [Test Example 5: Evaluation of DNA purification using various zeolites]

200 µL of a saliva specimen was added to 800 µL of a nucleic acid extraction reagent (0.5% SDS) and mixed, 240 mg of the zeolite described below was then added, and additionally mixed, the mixture was passed through a 0.45 µm filter, the zeolite and substances adsorbed thereon were removed, and the obtained solutions were used as samples for nucleic acid amplification (zeolite-treated samples 5-3 to 5-10). To 11.5 µL of a zeolite-treated sample, 11.5 µL of distilled water (DW) and 2 µL of the target nucleic acid solution described below were added, the mixture was reacted using a Simprova (registered trademark) Respiratory Infection Panel (commercially available from Eiken Chemical Co., Ltd.) at 64°C for 50 minutes, and the sample was subjected to target nucleic acid amplification according to an LAMP method. Next, the fluorescence value, which increases as a result of target nucleic acid amplification, was measured using a Thermal Cycler Dice (registered trademark) Real Time System III.

As a positive control, a sample 5-1 obtained by adding distilled water to the nucleic acid extraction reagent in place of a saliva specimen and simply mixing them was prepared, and as a negative control, a sample 5-2 (zeolite-untreated sample) obtained by adding a saliva specimen to the nucleic acid extraction reagent, but without adding a zeolite, followed by mixing was also prepared, and the samples were subjected to target nucleic acid amplification according to an LAMP method in the same manner.

The target nucleic acid solution used was Bordetella pertussis (B. pertussis) DNA (2,000 copies/test). The zeolites used are commercially available from Tosoh Corporation: model numbers 500KOA, 690HOA, 642NAA, 940NHA, 840NHA, 722HOA, 720NHA, and 720KOA. The results are shown in Table 11. Here, in Table 11, samples in which no detection occurred within the measurement time (50 minutes) are indicated as "N. D.".

**[Table 11]**

| Sample No. | Specimen | Model number | Crystal structure of zeolite | Cation in zeolite | Detection time (min) |
|---|---|---|---|---|---|
| 5-1 | no saliva (DW) | no zeolite treatment | | | 8.1 |
| 5-2 | saliva | no zeolite treatment | | | N.D. |
| 5-3 | saliva | 500KOA | L type | K | 8.9 |
| 5-4 | saliva | 690HOA | mordenite type | H | 12.8 |
| 5-5 | saliva | 642NAA | mordenite type | Na | 8.7 |
| 5-6 | saliva | 940NHA | beta type | NH₄ | 9.2 |
| 5-7 | saliva | 840NHA | ZSM-5 type | NH₄ | 13.9 |
| 5-8 | saliva | 722HOA | ferrierite type | H | 10.8 |
| 5-9 | saliva | 720NHA | ferrierite type | NH₄ | 9.4 |
| 5-10 | saliva | 720KOA | ferrierite type | K | 9.2 |

Comparing the sample 5-1 and the sample 5-2, in the sample 5-2, target nucleic acid amplification was not detected. This suggests that, when the saliva specimen was added, target nucleic acid amplification was inhibited, that is, there were substances other than nucleic acids (for example, a nucleic acid amplification inhibiting substance) in the saliva specimen. On the other hand, in the samples treated with the zeolite (samples 5-3 to 5-10), target nucleic acid amplification was confirmed, suggesting that substances other than nucleic acids in the saliva specimen were removed using the zeolite.

### [Test Example 6: Evaluation of RNA purification using various zeolites]

170 µL of a saliva specimen was added to 830 µL of a nucleic acid extraction reagent (0.5% SDS), and mixed, 240 mg of a zeolite was then added and additionally mixed, the mixture was passed through a 0.45 µm filter, the zeolite and substances adsorbed thereon were removed, and the obtained solutions were used as samples for nucleic acid amplification (zeolite-treated samples 6-3 to 6-10). To 9.5 µL of the zeolite-treated sample, 0.5 µL of a target nucleic acid solution was added, the mixture was reacted using a Loopamp (registered trademark) Novel Coronavirus 2019 (SARS-CoV-2) detection reagent kit (commercially available from Eiken Chemical Co., Ltd.) at 62.5°C for 90 minutes, and the sample was subjected to target nucleic acid amplification according to an LAMP method. Then, the turbidity value, which increases as a result of target nucleic acid amplification, was measured in real time using a LoopampEXIA Amplification Unit (commercially available from Eiken Chemical Co., Ltd.).

The target nucleic acid solution used was SARS-CoV-2 transcription RNA (250 copies/test). The zeolites used are commercially available from Tosoh Corporation: model numbers 500KOA, 690HOA, 642NAA, 940NHA, 840NHA, 722HOA, 720NHA, and 720KOA. As a positive control, a sample 6-1 obtained by adding distilled water to the nucleic acid extraction reagent in place of a saliva specimen and simply mixing them was also prepared, and subjected to target nucleic acid amplification according to an LAMP method in the same manner. In addition, as a negative control, a sample 6-2 (zeolite-untreated sample) obtained by adding a saliva specimen to the nucleic acid extraction reagent, but without adding a zeolite, followed by mixing was also subjected to target nucleic acid amplification according to the LAMP method in the same manner. The results are shown in Table 12.

**[Table 12]**

| Sample No. | Specimen | Model number | Crystal structure of zeolite | Cation in zeolite | Detection time (min) |
|---|---|---|---|---|---|
| 6-1 | no saliva (DW) | no zeolite treatment | | | 15.4 |
| 6-2 | saliva | no zeolite treatment | | | N.D. |
| 6-3 | saliva | 500KOA | L type | K | 26.6 |
| 6-4 | saliva | 690HOA | mordenite type | H | 21.6 |
| 6-5 | saliva | 642NAA | mordenite type | Na | 32.5 |
| 6-6 | saliva | 940NHA | beta type | NH₄ | 27.7 |
| 6-7 | saliva | 840NHA | ZSM-5 type | NH₄ | 23.9 |
| 6-8 | saliva | 722HOA | ferrierite type | H | 21.5 |
| 6-9 | saliva | 720NHA | ferrierite type | NH₄ | 27.9 |
| 6-10 | saliva | 720KOA | ferrierite type | K | 26.7 |

In the same manner as in Test Example 5, comparing the samples 6-1 and 6-2, in the sample 6-1, target nucleic acid amplification was detected, but in the sample 6-2, target nucleic acid amplification was not detected, suggesting that there were substances other than nucleic acids (for example, a nucleic acid amplification inhibiting substance) in the saliva specimen. On the other hand, in the samples treated with the zeolite (samples 6-3 to 6-10), target nucleic acid amplification was confirmed, suggesting that substances other than nucleic acids in the saliva specimen were removed using the zeolite.

### [Test Example 7: Application of DNA purified using aprotic zeolite to PCR]

200 µL of a solution obtained by adding Streptococcus pneumoniae (S. pneumoniae) to a saliva specimen was mixed with 800 µL of a nucleic acid extraction reagent (0.5% SDS), 240 mg of a zeolite (model number 720NHA, commercially available from Tosoh Corporation) was added, and the mixture was mixed by inversion. The suspension mixed by inversion was passed through a 0.45 µm filter, and the zeolite and substances adsorbed thereon were removed to obtain a sample 7-4. The target nucleic acid was amplified using 5 µL of the obtained sample 7-4 and 20 µL of the quantitative PCR reaction reagent according to the quantitative PCR method. In addition, at the same time, a sample 7-3 obtained by mixing a solution in which S. Pneumoniae was added to saliva with a nucleic acid extraction reagent and not treating it with a zeolite, a sample 7-1 obtained by mixing a solution in which S. pneumoniae was added to saline with a nucleic acid extraction reagent and not treating it with a zeolite, and a sample 7-2 obtained by treating the resulting saline-based mixture with a zeolite were also prepared, and the samples were subjected to target nucleic acid amplification according to the quantitative PCR method in the same manner.

Amplification and detection of the target nucleic acid according to the quantitative PCR method were performed using the primer and probe set shown in Table 13, according to the reaction under the temperature and time shown in Table 15 in the quantitative PCR reaction reagent solution having the composition shown in Table 14, and the fluorescence value, which increases as a result of target nucleic acid amplification, was measured in real time using a LightCycler (registered trademark) 480 System. Here, the probe composed of a base sequence shown in SEQ ID NO 9 in Table 13 was labeled with FAM at the 5' end and labeled with TAMRA at the 3' end. The results are shown in Table 16. Here, in Table 16, the "proportion of copies" is the percentage relative to the number of copies of the sample 7-1 (defined as 100%).

**[Table 13]**

| <Primer and probe set for quantitative PCR method targeting S. *pneumoniae* DNA as nucleic acid> | |
|---|---|
| Name | Sequence (5'→3') |
| Forward primer | ACGCAATCTAGCAGATGAAGC (SEQ ID NO 7) |
| Reverse primer | TGTTTGGTTGGTTATTCGTGC (SEQ ID NO 8) |
| Probe | FAM-TTTGCCGAAAACGCTTGATACAGGG (SEQ ID NO 9)-TAMRA |

**[Table 14]**

| <Composition of quantitative PCR reaction reagent (20 µL)> |
|---|
| 1× Premix Ex Taq (Probe qPCR) |
| 0.2 µM forward primer |
| 0.2 µM reverse Primer |
| 0.2 µM probe |
| Distilled water |

**[Table 15]**

| <Reaction conditions> | | |
|---|---|---|
| Step 1 | 95°C 30 sec | 1 cycle |
| Step 2 | 95°C 5 sec→60°C 30 sec | 40 cycles |

**[Table 16]**

| Sample No. | Specimen | Zeolite | Number of copies (copies/test) | Proportion of copies |
|---|---|---|---|---|
| 7-1 | bacteria+saline | no zeolite treatment | 107,243 | 100% |
| 7-2 | bacteria+saline | with zeolite treatment | 105,363 | 98% |
| 7-3 | bacteria+saliva | no zeolite treatment | 74,135 | 69% |
| 7-4 | bacteria+saliva | with zeolite treatment | 102,129 | 95% |

The numbers of target nucleic acid copies of the samples 7-1 and 7-2 were similar. This result suggests that S. pneumoniae was extracted using the nucleic acid extraction reagent, and the nucleic acid (DNA) of S. pneumoniae was not adsorbed to the zeolite, but remained detectable. Comparing the samples 7-3 and 7-4, in the sample 7-3 not treated with the aprotic zeolite, the number of target nucleic acid copies detected was lower compared to the samples 7-1 and 7-2 to which no saliva specimen was added. On the other hand, in the sample treated with the aprotic zeolite 7-4, the number of target nucleic acid copies detected was comparable to that of the sample to which no saliva specimen was added. This suggests that, when the aprotic zeolite treatment was performed, it was possible to remove substances other than nucleic acids (for example, a nucleic acid amplification inhibiting substance) contained in the saliva specimen. That is, it suggests that nucleic acids could be extracted and purified with high purity.

### [Test Example 8: Application of RNA purified using aprotic zeolite to PCR]

200 µL of a solution obtained by adding influenza A virus to a different saliva specimen 1 or 2 and 800 µL of a nucleic acid extraction reagent (0.5% SDS) were mixed, 240 mg of a zeolite (model number 720NHA, commercially available from Tosoh Corporation) was added, and the mixture was mixed by inversion. The suspension mixed by inversion was passed through a 0.45 µm filter, and the zeolite and substances adsorbed thereon were removed to obtain samples 8-4 and 8-6. The target nucleic acid was amplified using 5 µL of the obtained samples 8-4 and 8-6 and 20 µL of the quantitative PCR reaction reagent according to the quantitative PCR method. In addition, at the same time, samples 8-3 and 8-5 obtained without the zeolite treatment, a sample 8-1, which was obtained by mixing a solution of influenza A virus to saline and a nucleic acid extraction reagent and not treated with the zeolite, and a sample 8-2 obtained with the zeolite treatment were also subjected to target nucleic acid amplification according to the quantitative PCR method in the same manner.

Amplification of the target nucleic acid according to the quantitative PCR method was performed using the primer set shown in Table 17, according to the reaction under the temperature and time shown in Table 3 in the quantitative PCR reaction reagent solution having the composition shown in Table 2, and the fluorescence value, which increases as a result of target nucleic acid amplification was measured in real time using a LightCycler (registered trademark) 480 System. Here, the probe composed of a base sequence shown in SEQ ID NO 12 in Table 17 was labeled with FAM at the 5' end and labeled with MGB at the 3' end. The results are shown in Table 18. Here, in Table 18, the "proportion of copies" is the percentage relative to the number of copies of the sample 8-1 (defined as 100%).

**[Table 17]**

| <Primer and probe set for quantitative PCR method targeting influenza A virus RNA as nucleic acid> | |
|---|---|
| Name | Sequence (5'→3') |
| Forward primer | CCMAGGTCGAAACGTAYGTTCTCTCTATC (SEQ ID NO 10) |
| Reverse primer | TGACAGRATYGGTCTTGTCTTTAGCCAYTCCA (SEQ ID NO 11) |
| Probe | FAM-CAGCCAGCAATRTTRCATTTACC (SEQ ID NO 12)-MGB |

**[Table 18]**

| Sample No. | Specimen | Zeolite treatment | Number of copies (copies/test) | Proportion of copies |
|---|---|---|---|---|
| 8-1 | virus+saline | no zeolite treatment | 255,006 | 100% |
| 8-2 | virus+saline | with zeolite treatment | 257,304 | 101% |
| 8-3 | virus+saliva 1 | no zeolite treatment | 156,584 | 61% |
| 8-4 | virus+saliva 1 | with zeolite treatment | 270,475 | 106% |
| 8-5 | virus+saliva 2 | no zeolite treatment | 153,701 | 60% |
| 8-6 | virus+saliva 2 | with zeolite treatment | 263,219 | 103% |

The results showing that the numbers of target nucleic acid copies of the samples 8-1 and 8-2 were comparable suggest that influenza A virus was extracted by the nucleic acid extraction reagent and that the nucleic acid (RNA) of influenza A virus was not adsorbed to the zeolite and was detected. Compared with the samples 8-1 and 8-2 to which no saliva specimen was added, the number of target nucleic acid copies detected was reduced by half in the sample 8-3 or sample 8-5 not treated with the aprotic zeolite. On the other hand, in the samples 8-4 and 8-6, which were treated with an aprotic zeolite, the number of target nucleic acid copies detected was comparable to that of the sample to which no saliva was added. This suggests that, when the aprotic zeolite treatment was performed, it was possible to remove substances other than nucleic acids (for example, a nucleic acid amplification inhibiting substance) contained in the saliva specimen. That is, it suggests that nucleic acids could be purified with high purity.

### [Test Example 9: RNA purification using aprotic zeolite with other specimens]

A nucleic acid extraction reagent (0.5% SDS) was mixed with each of various specimens (urine, cerebrospinal fluid, nasal swab, nasopharyngeal swab, serum, whole blood, feces, and sputum), 240 mg of the zeolite described below was then added, and additionally mixed, the mixture was passed through a 0.45 µm filter, the zeolite and substances adsorbed thereon were removed, and the obtained solution was used as a sample for nucleic acid amplification (zeolite-treated sample), To 9.5 µL of a zeolite-treated sample , 0.5 µL of a target nucleic acid solution was added, the mixture was reacted using a Loopamp (registered trademark) Novel Coronavirus 2019 (SARS-CoV-2) detection reagent kit (commercially available from Eiken Chemical Co., Ltd.) at 62.5°C for 35 minutes, and the turbidity value, which increases as a result of target nucleic acid amplification, was measured in real time using a LoopampEXIA Amplification Unit (commercially available from Eiken Chemical Co., Ltd.).

The target nucleic acid solution used was SARS-CoV-2 transcription RNA (250 copies/test). The zeolites used are commercially available from Tosoh Corporation: model numbers 690HOA and 720NHA. As a positive control, a sample obtained by adding distilled water to the nucleic acid extraction reagent in place of a saliva specimen and mixing them was also prepared, and the target nucleic acid was amplified in the same manner according to an LAMP method. In addition, as a negative control, a sample obtained by adding a saliva specimen to a nucleic acid extraction reagent, but without adding a zeolite, followed by mixing (zeolite-untreated sample) was also subjected to target nucleic acid amplification according to an LAMP method in the same manner The results are shown in Tables 19 to 26.

**[Table 19]**

| Specimen | Zeolite | Detection time (min) |
|---|---|---|
| DW | untreated | 16.6 |
| Urine | untreated | N.D. |
| | 720NHA | 27.9 |
| | 690HOA | N.D. |

**[Table 20]**

| Specimen | Zeolite | Detection time (min) |
|---|---|---|
| DW | untreated | 17.6 |
| Cerebrospinal fluid | untreated | N.D. |
| | 720NHA | 24.0 |
| | 690HOA | 26.8 |

**[Table 21]**

| Specimen | Zeolite | Detection time (min) |
|---|---|---|
| DW | untreated | 16.1 |
| Nasal swab | untreated | N.D. |
| | 720NHA | 32.8 |
| | 690HOA | N.D. |

**[Table 22]**

| Specimen | Zeolite | Detection time (min) |
|---|---|---|
| DW | untreated | 15.0 |
| Nasopharyngeal swab | untreated | N.D. |
| | 720NHA | 27.7 |
| | 690HOA | N.D. |

**[Table 23]**

| Specimen | Zeolite | Detection time (min) |
|---|---|---|
| DW | untreated | 17.2 |
| Serum | untreated | N.D. |
| | 720NHA | 26.6 |
| | 690HOA | N.D. |

**[Table 24]**

| Specimen | Zeolite | Detection time (min) |
|---|---|---|
| DW | untreated | 17.5 |
| Whole blood | untreated | N.D. |
| | 720NHA | 24.4 |
| | 690HOA | N.D. |

**[Table 25]**

| Specimen | Zeolite | Detection time (min) |
|---|---|---|
| DW | untreated | 17.7 |
| Feces | untreated | N.D. |
| | 720NHA | 23.5 |
| | 690HOA | 20.4 |

**[Table 26]**

| Specimen | Zeolite | Detection time (min) |
|---|---|---|
| DW | untreated | 15.9 |
| Sputum | untreated | N.D. |

| | | |
|---|---|---|
| | 720NHA | 22.3 |
| | 690HOA | 27.3 |

In the zeolite-untreated samples, target nucleic acids were not detected or detection times were significantly delayed compared to the samples to which no specimen was added. This is thought to be because substances other than nucleic acids contained in various specimens were not removed. On the other hand, in the zeolite-treated samples, target nucleic acids were detectable. This is thought to be because substances other than nucleic acids contained in various specimens were removed using the zeolite. Among the various specimens, higher effectiveness was confirmed with the aprotic zeolite than with the proton-type zeolite for urine, nasal swab, nasopharyngeal swab, serum and whole blood.

### [Test Example 10: Evaluation of nucleic acid purification depending on type of surfactant in nucleic acid extraction reagent]

890 µL of a nucleic acid extraction reagent containing various surfactants shown in Table 27 and 110 µL of a saliva specimen were mixed, 240 mg of a zeolite (model number 720NHA, commercially available from Tosoh Corporation) was then added, and additionally mixed, and the mixture was passed through a 0.45 µm filter, and the zeolite and substances adsorbed thereon were removed to obtain samples 10-20 to 10-28. The obtained samples 10-20 to 10-28 were used as samples for nucleic acid amplification (aprotic zeolite-treated samples). To 9.5 µL of a zeolite-treated sample , 0.5 µL of a target nucleic acid solution was added, the mixture was reacted using a Loopamp (registered trademark) Novel Coronavirus 2019 (SARS-CoV-2) detection reagent kit (commercially available from Eiken Chemical Co., Ltd.) at 62.5°C for 35 minutes, and the turbidity value, which increases according target nucleic acid amplification, was measured in real time using a LoopampEXIA Amplification Unit (commercially available from Eiken Chemical Co., Ltd.).

The target nucleic acid solution used was SARS-CoV-2 transcription RNA (250 copies/test). As positive controls, samples 10-1 to 10-10 obtained by adding distilled water to various nucleic acid extraction reagents in place of a saliva specimen and mixing them were also prepared, and subjected to target nucleic acid amplification according to an LAMP method in the same manner. In addition, as negative controls, samples 10-11 to 10-19 obtained by adding a saliva specimen to a nucleic acid extraction reagent, but without adding a zeolite, followed by mixing (zeolite-untreated sample) were subjected to target nucleic acid amplification according to an LAMP method in the same manner. The results are shown in Table 27.

**[Table 27]**

| Sample No. | Specimen | Zeolite treatment | Type of surfactant | Detection time (min) |
|---|---|---|---|---|
| 10-1 | DW | no | no surfactant | 16.8 |
| 10-2 | DW | no | sodium dodecyl sulfate | 16.4 |
| 10-3 | DW | no | lithium dodecyl sulfate | 16.2 |
| 10-4 | DW | no | sodium cholate | 19.2 |
| 10-5 | DW | no | benzalkonium chloride | 16.4 |
| 10-6 | DW | no | TRITON (registered | 16.2 |
| | | | trademark) X-100 | |
| 10-7 | DW | no | Tween (registered trademark) 40 | 15.6 |
| 10-8 | DW | no | CHAPSO | 18.0 |
| 10-9 | DW | no | CHAPS | 18.2 |
| 10-10 | DW | no | NDSB | 15.6 |
| 10-11 | saliva | no | sodium dodecyl sulfate | N.D. |
| 10-12 | saliva | no | lithium dodecyl sulfate | N.D. |
| 10-13 | saliva | no | sodium cholate | N.D. |
| 10-14 | saliva | no | benzalkonium chloride | N.D. |
| 10-15 | saliva | no | TRITON (registered trademark) X-100 | N.D. |
| 10-16 | saliva | no | Tween (registered trademark) 40 | N.D. |
| 10-17 | saliva | no | CHAPSO | N.D. |
| 10-18 | saliva | no | CHAPS | N.D. |
| 10-19 | saliva | no | NDSB | N.D. |
| 10-20 | saliva | 720NHA | sodium dodecyl sulfate | 18.6 |
| 10-21 | saliva | 720NHA | lithium dodecyl sulfate | 18.7 |
| 10-22 | saliva | 720NHA | sodium cholate | 18.4 |
| 10-23 | saliva | 720NHA | benzalkonium chloride | 17.1 |
| 10-24 | saliva | 720NHA | TRITON (registered trademark) X-100 | 15.3 |
| 10-25 | saliva | 720NHA | Tween (registered trademark) 40 | 16.2 |
| 10-26 | saliva | 720NHA | CHAPSO | 16.5 |
| 10-27 | saliva | 720NHA | CHAPS | 15.9 |
| 10-28 | saliva | 720NHA | NDSB | 15.8 |

In the samples 10-11 to 10-19 (zeolite-untreated samples) obtained by simply mixing a saliva specimen with a nucleic acid extraction reagent, the target nucleic acid was not detected. On the other hand, regardless of which surfactant was contained in the nucleic acid extraction reagent, target nucleic acid amplification was detected in the aprotic zeolite-treated samples 10-20 to 10-28. These results indicate that, regardless of which surfactant was contained in the nucleic acid extraction reagent, substances other than nucleic acids (for example, a nucleic acid amplification inhibiting substance) contained in the saliva specimen could be removed using the aprotic zeolite.

## Claims

1. A nucleic acid purification method, comprising a step of bringing a sample containing a nucleic acid into contact with an aprotic zeolite.

2. The purification method according to claim 1, wherein the sample is derived from at least one specimen selected from the group consisting of blood, cerebrospinal fluid, urine, feces, sputum, saliva, nasal discharge, swab fluid, amniotic fluid, and gargle fluid.

3. The purification method according to claim 1 or 2, wherein, in the contacting step, at least a portion of the nucleic acid is not adsorbed to the aprotic zeolite.

4. The purification method according to claim 1 or 2, wherein the aprotic zeolite is at least one selected from the group consisting of a ferrierite-type zeolite, a ZSM-5-type zeolite, a Y-type zeolite, and a beta-type zeolite.

5. The purification method according to claim 4, wherein the aprotic zeolite is at least one selected from the group consisting of a ferrierite-NH₄-type zeolite, a ferrierite-K-type zeolite, a ZSM-5-NH₄-type zeolite, a Y-Na-type zeolite, and a beta-NH₄-type zeolite.

6. The purification method according to claim 2, further comprising,
before the contacting step, a step of obtaining a sample containing the nucleic acid by mixing the specimen with a nucleic acid extraction reagent containing a surfactant and/or by heating the specimen.

7. The purification method according to claim 6, wherein the surfactant includes an anionic surfactant.

8. The purification method according to claim 7, wherein the anionic surfactant is dodecyl sulfate.

9. The purification method according to claim 1 or 2, wherein the nucleic acid is RNA and/or DNA.

10. A kit for purifying a nucleic acid, comprising an aprotic zeolite.

11. The kit according to claim 10, wherein the aprotic zeolite is at least one selected from the group consisting of a ferrierite-type zeolite, a ZSM-5-type zeolite, a Y-type zeolite, and a beta-type zeolite.

12. The kit according to claim 11, wherein the aprotic zeolite is at least one selected from the group consisting of a ferrierite-NH₄-type zeolite, a ferrierite-K-type zeolite, a ZSM-5-NH₄-type zeolite, a Y-Na-type zeolite, and a beta-NH₄-type zeolite.

13. The kit according to claim 10, wherein the kit is a kit for purifying a nucleic acid derived from at least one specimen selected from the group consisting of blood, cerebrospinal fluid, urine, feces, sputum, saliva, nasal discharge, swab fluid, amniotic fluid, and gargle fluid, and further includes a nucleic acid extraction reagent containing a surfactant.

14. The kit according to claim 13, wherein the surfactant includes an anionic surfactant.

15. The kit according to claim 14, wherein the anionic surfactant is dodecyl sulfate.
